# EUROPÄISCHE PATENTSCHRIFT

(11) **EP 3 610 013 B1**
(45) Veröffentlichungstag und Bekanntmachung des Hinweises auf die Patenterteilung: **03.04.2024**
(21) Anmeldenummer: 18716238.3
(22) Anmeldetag: 05.04.2018
(51) Int. Cl.: C12Q 1/6806, B01L 3/00

(54) **DESORPTION VON NUKLEINSÄUREN**
DESORPTION OF NUCLEIC ACIDS
DÉSORPTION D'ACIDES NUCLÉIQUES

(30) Priorität: 11.04.2017 DE 102017206155
(43) Veröffentlichungstag der Anmeldung: 19.02.2020
(73) Patentinhaber: Robert Bosch GmbH, 70442 Stuttgart (DE)
(72) Erfinder: RUPP, Jochen, 70569 Stuttgart (DE); DORRER, Christian, 71364 Winnenden (DE); STEIGERT, Juergen, 70176 Stuttgart (DE); FALTIN, Bernd, 70839 Gerlingen (DE); SEIDL, Karsten, 70839 Gerlingen (DE)
(86) Internationale Anmeldenummer: PCT/EP2018/058727
(87) Internationale Veröffentlichungsnummer: WO 2018/189025

(56) Entgegenhaltungen:
- WO-A1-2015/188994
- WO-A2-2008/002725
- US-A1- 2012 214 168
- US-B1- 9 322 014
- D. D. MAMAEV ET AL: "Method for automated extraction and purification of nucleic acids and its implementation in microfluidic system", APPLIED BIOCHEMISTRY AND MICROBIOLOGY, Bd. 47, Nr. 2, 1. März 2011 (2011-03-01), Seiten 211-220, XP055476599, US ISSN: 0003-6838, DOI: 10.1134/S0003683811020128
- ATHINA S. KASTANIA ET AL: "Plasma micro-nanotextured polymeric micromixer for DNA purification with high efficiency and dynamic range", ANALYTICA CHIMICA ACTA, Bd. 942, 1. Oktober 2016 (2016-10-01), Seiten 58-67, XP055476608, AMSTERDAM, NL ISSN: 0003-2670, DOI: 10.1016/j.aca.2016.09.007
- BREADMORE MICHAEL C ET AL: "Microchip-based purification of DNA from biological samples", ANALYTICAL CHEMISTRY, AMERICAN CHEMICAL SOCIETY, US, Bd. 75, Nr. 8, 15. April 2003 (2003-04-15) , Seiten 1880-1886, XP002460392, ISSN: 0003-2700, DOI: 10.1021/AC0204855

## Beschreibung

Die Erfindung betrifft ein Verfahren zur Desorption von Nukleinsäuren aus einer Probe.

### Stand der Technik

Aus der deutschen Offenlegungsschrift DT 23 39 111 A1 ist ein Verfahren zur Reinigung von Proteinen und anderen biospezifisch adsorbierbaren Substanzen durch biospezifische Adsorption und durch Desorption bekannt, wobei die Adsorption an Ultrafiltrationsmembranen erfolgt. Aus der europäischen Patentschrift EP 0 700 521 B1 ist eine Sonde bekannt, die in einem Massenspektrometer entnehmbar eingebracht werden kann, wobei die Sonde eine probenpräsentierende Oberfläche für das Präsentieren eines Analyts gegenüber einer Energiequelle, die eine für das Desorbieren/lonisieren des Analyts von der Sonde für den Analytnachweis geeignete Energie abgibt, aufweist, wobei die Oberfläche mit einem Affinitätsreagens derivatisiert ist, welches den Analyt binden kann und aus Metallionen, Nukleinsäuren, Peptiden, Kohlenhydraten, Proteinen und Kombinationen derselben gewählt wird, wobei ein desorptions/ionisationsunterstützendes Matrixmaterial an der Oberfläche in Assoziation mit dem Affinitätsreagens vorgesehen wird. Das Dokument US 9 322 014 B1 beschreibt beispielsweise ein Verfahren zur Desorption von Nukleinsäuren aus einer Probe.

### Offenbarung der Erfindung

Das Verfahren zur Desorption von Nukleinsäuren aus einer Probe ist dadurch gekennzeichnet, dass eine Festphase in einem mikrofluidischen System mehrfach mit einem Elutionspuffer gespült wird, um an die Festphase gebundene Nukleinsäuren in dem mikrofluidischen System von der Festphase zu eluieren.

Bei der Probe handelt es sich vorzugsweise um eine lysierte Probe, zum Beispiel um eine Flüssigkeit, welche Zielzellen enthält, zum Beispiel eine Zellsuspension oder eine Patientenprobe, wie Blut, Lavage, Sputum oder ein ausgespülter Swab beziehungsweise Abstrich. Die Probe hat zum Beispiel ein Volumen zwischen einigen Mikrolitern und zehn Millilitern. Typischerweise hat die Probe ein Volumen zwischen einem halben und einem Milliliter. Bei der Festphase handelt es sich vorzugsweise um einen Filter, zum Beispiel einen Silika-Filter. Bei dem Elutionspuffer handelt es sich zum Beispiel um Wasser oder um Wasser mit geeigneten Zusätzen. Die Festphase kann auch als stationäre Phase bezeichnet werden. Analog kann der Elutionspuffer, der auch als Eluent bezeichnet wird, auch als mobile Phase bezeichnet werden. Zur Gewinnung von Nukleinsäuren aus Zellen werden diese oftmals zunächst lysiert und die freigesetzten Nukleinsäuren in einem anschließenden Schritt aufgereinigt. Die Nukleinsäuren werden dann weiterverarbeitet oder analysiert. Beispielsweise können die aufgereinigten Nukleinsäuren mittels einer PCR selektiv amplifiziert werden. Dabei stehen die Großbuchstaben PCR für die englischen Begriffe Polymerase Chain Reaction. Das entspricht im Deutschen einer Polymerase-Kettenreaktion. Zur Lyse von Zellen gibt es verschiedene Verfahren, zum Beispiel kann diese chemisch, enzymatisch oder mechanisch erfolgen. Zur Aufreinigung der aus den Zellen freigesetzten Nukleinsäuren wird das durch die Lyse entstandene Lysat oftmals mit einem Bindepuffer versetzt und mit einer festen Matrix, zum Beispiel einem Silika-Filter, in Kontakt gebracht, wobei die Nukleinsäuren am Filter adsorbieren. Die Nukleinsäuren können dann gewaschen und eluiert werden. Dieses Eluat enthält Nukleinsäuren und kann in weiteren enzymatischen Reaktionen verwendet werden, beispielsweise PCR, Sequenzierung oder Restriktionsenzymverdau. Durch das Prozessieren in dem mikrofluidischen System kann eine unerwünscht hohe Konzentration inhibitorischer Bestandteile in einem Eluat reduziert werden. Mikrofluidische Systeme sind an sich bekannt, zum Beispiel aus der deutschen Offenlegungsschrift DE 10 2009 028 496 A1. Durch die Durchführung des beanspruchten Verfahrens in dem mikrofluidischen System können einzelne Fraktionen eines Eluats besonders verlustarm hergestellt werden. Als Fraktion wird ein definiertes Volumen bezeichnet, das beispielsweise durch eine in das mikrofluidische System integrierte Mikropumpe in ein ebenfalls in das mikrofluidische System integriertes Kanalsystem verdrängt wird. Die einzelnen Fraktionen unterscheiden sich in Konzentration von Nukleinsäuren und gegebenenfalls vorhandener Inhibitoren. Durch eine geeignete Prozessführung können gegebenenfalls Fraktionen mit hoher Inhibitorenkonzentration verworfen werden. Dann kann für eine anschließende Analyse des Eluats vorteilhaft eine Fraktion verwendet werden, deren Inhibitorenkonzentration geringer ist. Durch das mehrmalige Durchströmen der Festphase mit dem Elutionspuffer kann sich ein Sättigungsgleichgewicht einstellen, bei dem die maximale Elutionseffizienz erreicht wird.

Ein bevorzugtes Ausführungsbeispiel des Verfahrens ist dadurch gekennzeichnet, dass die Elution durch eine in das mikrofluidische System integrierte Mikropumpe mit einem definierten Verdrängungsvolumen erfolgt, das einem Elutionsvolumen entspricht. Die Elution erfolgt vorteilhaft durch ein definiertes Volumen des Elutionspuffers, das durch die Mikropumpe verdrängt und über die Festphase geleitet wird. Das definierte Volumen wird als Elutionsvolumen bezeichnet. Der Prozess und das Volumen des mikrofluidischen Kanalnetzes oder Netzwerks werden vorteilhaft genau aufeinander abgestimmt. Die Volumina von einzelnen Fraktionen können sich auch unterscheiden. So kann zum Beispiel in einem ersten Schritt mit einer kleinen Pumpe (5 bis 15 Mikroliter pro Hub; typischerweise 11 Mikroliter) verwendet werden, um den Waschpuffer, der noch im Bereich des Filters und der zu- beziehungsweise abführenden Kanäle vorhanden ist, zu verdrängen. Für den Transport des Eluats in nachfolgende Prozesskammern, zum Beispiel für eine PCR, wird jedoch ein größeres Volumen benötigt, welches mit einer Pumpe mit größerer Pumpkammer (10 bis 25 Mikroliter; typischerweise 20 Mikroliter) realisiert werden kann. Das Volumen pro Elutionsfraktion liegt zwischen 10 bis 100 Mikroliter, insbesondere zwischen 15 bis 50 Mikroliter, beispielsweise 20 Mikroliter. In einer vorteilhaften Ausführung werden zum Pumpen der Flüssigkeiten im mikrofluidischen System Mikromembranpumpen verwendet, deren Pumpkammervolumen gerade dem Volumen einer Elutionsfraktion entspricht. Dies hat den Vorteil, dass bei jedem Pumphub gerade das Volumen einer Elutionfraktion über den Filter geschoben wird und das Volumen somit besonders genau eingestellt werden kann.

Ein weiteres bevorzugtes Ausführungsbeispiel des Verfahrens ist dadurch gekennzeichnet, dass das Elutionsvolumen ein bis einhundert Mikroliter beträgt. Das Elutionsvolumen kann nur einige Mikroliter betragen. Das Elutionsvolumen beträgt bevorzugt fünfzehn bis fünfzig Mikroliter. Besonders bevorzugt beträgt das Elutionsvolumen zirka zwanzig Mikroliter. Mit diesem Elutionsvolumen wurden bei im Rahmen der vorliegenden Erfindung durchgeführten Versuchen und Untersuchungen die besten Resultate erzielt.

Ein weiteres bevorzugtes Ausführungsbeispiel des Verfahrens ist dadurch gekennzeichnet, dass ein Elutionsmedium in dem mikrofluidischen System über den Filter hin- und hergepumpt wird. Das liefert unter anderem den Vorteil, dass eine besonders gute Durchmischung der Elutionsfraktion erfolgt und Nukleinsäuren besonders gut, insbesondere vollständig, vom Filter gelöst werden.

Ein weiteres bevorzugtes Ausführungsbeispiel des Verfahrens ist dadurch gekennzeichnet, dass eine Anströmrichtung der Festphase in dem mikrofluidischen System umgekehrt wird. So kann ein Elutionsvolumen mehrmals über dieselbe Festphase geleitet werden. Je nach Ausführung des mikrofluidischen Systems kann ein Elutionsvolumen aber auch unidirektional, also nur in einer Richtung, mehrmals über die Festphase geleitet werden. Beide Verfahren, also sowohl mit einer unidirektionalen als auch mit einer bidirektionalen Anströmung der Festphase, liefern unter anderem den Vorteil, dass sich Nukleinsäure dem Konzentrationsgradienten folgend in einer Fraktion anreichern können.

Erfindungsgemäß werden Teilvolumina des Elutionsvolumens seriell nacheinander über die Festphase beziehungsweise den Filter geleitet. Das hat den Vorteil, dass in jeder weiteren Eluatfraktion die Konzentration an inhibitorischen Substanzen reduziert wird, da diese bereits durch die vorgehende Eluatfraktion verdrängt wird. Typischerweise nimmt jedoch die Ausbeute an Nukleinsäuren mit jedem weiteren Elutionsschritt ab, wodurch in der ersten Fraktion die höchste Konzentration an Nukleinsäuren und inhibitorischer Substanzen erwartet wird. Daher wird die erste Eluatfraktion verworfen.

Die erste Fraktion des Elutionspuffers kann vorteilhaft verwendet werden, um die Reste des Waschpuffers zu verdrängen. Diese erste Fraktion des Elutionspuffers kann zum Beispiel in einer Auffangkammer innerhalb des mikrofluidischen Netzwerks oder Kanalnetzes des mikrofluidischen Systems gepumpt werden. Das Fraktionsvolumen ist hierbei im Idealfall ähnlich, zum Beispiel ein Vielfaches eines Totvolumens. Als Totvolumen wird ein freies Volumen in der Festphase und in einem Kanal des fluidischen Systems bezeichnet, der mit Waschpuffer gefüllt ist. Anschließend kann eine zweite Fraktion ein- oder mehrmals über die Festphase geleitet werden, um die Ausbeute der Nukleinsäure zu erhöhen.

Ein weiteres bevorzugtes Ausführungsbeispiel des Verfahrens ist dadurch gekennzeichnet, dass eine erste Eluatfraktion und eine zweite Eluatfraktion in einer mikrofluidischen Kammer in dem mikrofluidischen System in einem Gemisch vereint werden, aus dem ein Aliquot für die weitere Analyse entnommen wird. Als Aliquot wird eine Teilportion des Gemischs bezeichnet. Durch den Vermischungsschritt werden die Konzentrationen der inhibitorischen Substanzen und der Nukleinsäure reduziert. Die Konzentration der inhibitorischen Substanzen kann durch dieses Verfahren beispielsweise soweit reduziert werden, dass eine empfindliche, enzymatische Nachweisreaktion nun überhaupt erst erfolgreich durchgeführt werden kann. Dies ist insofern vorteilhaft, da durch ein geeignetes enzymatisches Nachweisverfahren, zum Beispiel PCR, selbst geringste Mengen an Nukleinsäure amplifiziert werden können, wodurch die zunächst nachteilig wirkende Reduktion der Nukleinsäurekonzentration im entnommenen Aliquot sich als vorteilhaft erweist.

Ein weiteres bevorzugtes Ausführungsbeispiel des Verfahrens ist dadurch gekennzeichnet, dass eine Eluatfraktion n und eine Eluatfraktion n + 1 in dem mikrofluidischen System in einem Gemisch vereint werden. So können zum Beispiel eine dritte und eine vierte Eluatfraktion in einem Gemisch vereint werden. Der Buchstabe n steht für eine natürliche Zahl größer als 1.

Ein weiteres bevorzugtes Ausführungsbeispiel des Verfahrens ist dadurch gekennzeichnet, dass vor der Elution mindestens einer, mehrere oder jeder der folgenden vorbereitenden Schritte in dem mikrofluidischen System ausgeführt wird beziehungsweise werden: Die Probe wird vor der Elution in dem mikrofluidischen System lysiert, um die Zielzellen zu lysieren und die Nukleinsäuren freizusetzen; dem Lysat wird in dem mikrofluidischen System in einem Bindeschritt, der optional ist, ein Bindepuffer hinzugegeben, der mit dem Lysat vermischt wird; das Lysat beziehungsweise das in dem Bindeschritt entstandene Gemisch wird in dem mikrofluidischen System über die Festphase geleitet. Dabei erfolgt eine Adsorption der Nukleinsäuren an die Festphase. Typischerweise enthalten die Bindepuffer einen hohen Anteil chaotroper Substanzen, beispielsweise Guanidinthiocyanat, Guanidinhydrochlorid oder Cäsiumchlorid und/oder Alkohol, beispielsweise Ethanol oder Isopropanol. Diese Substanzen sind bekannt dafür, dass sie enzymatische downstream Anwendungen, wie beispielsweise PCR, inhibieren oder gegebenenfalls vollständig zum Erliegen bringen. Zudem bringt das lysierte Probenmaterial Substanzen mit, die eine anschließende enzymatische Prozessierung der Nukleinsäure stören können. Aus diesem Grund wird die Festphase mit der adsorbierten Nukleinsäure mit einem Waschpuffer umspült, der die störenden Bestandteile von der Festphase entfernt oder deren Konzentration reduziert. Diese Waschpuffer enthalten typischerweise chaotrope Substanzen, typischerweise in geringerer Konzentration als der Bindepuffer, und/oder Alkohole. Durch die beanspruchte fluidische Prozessführung in dem mikrofluidischen System kann die Konzentration der inhibitorischen Bestandteile in dem Eluat wirksam reduziert werden.

Die Erfindung betrifft des Weiteren ein Computerprogrammprodukt mit einem Computerprogramm, das Softwaremittel zum Durchführen eines vorab beschriebenen Verfahrens aufweist, wenn das Computerprogramm auf einem Computer ausgeführt wird. Der Computer gehört vorzugsweise zu einer mikrofluidischen Plattform und dient vorteilhaft zur Steuerung der Prozessführung in dem mikrofluidischen System.

Die oben angegebene Aufgabe ist bei einem mikrofluidischen System mit einem Kanalnetz, in welchem eine Festphase und mindestens eine Mikropumpe angeordnet sind, alternativ oder zusätzlich dadurch gelöst, dass die Mikropumpe so in dem Kanalnetz angeordnet und dazu eingerichtet ist, die Festphase in dem mikrofluidischen System mehrfach mit einem Elutionspuffer zu spülen, um Nukleinsäuren aus einer vorzugsweise lysierten Probe, insbesondere gemäß einem vorab beschriebenen Verfahren, zu desorbieren. Die Mikropumpe ist zum Beispiel als Mikromembranpumpe ausgeführt, wie sie in der deutschen Offenlegungsschrift DE 10 2010 001 410 A1 beschrieben ist. Ein Pumpenkammervolumen der Mikropumpe, insbesondere Mikromembranpumpe, entspricht einem Volumen einer Eluatfraktion. Das mikrofluidische System umfasst vorteilhaft einen Polymerchip, der ein Lab-on-Chip darstellt, wie er in der deutschen Offenlegungsschrift DE 10 2011 085 371 A1 offenbart ist.

Ein bevorzugtes Ausführungsbeispiel des mikrofluidischen Systems ist dadurch gekennzeichnet, dass die Festphase ein in dem Kanalnetz des mikrofluidischen Systems angeordneter Filter ist, der über einen Verbindungskanal mit der Mikropumpe verbunden ist. Bei dem Filter handelt es sich zum Beispiel um einen Silika-Filter. Die Mikropumpe ist vorteilhaft zwischen einem Vorratsreservoir und dem Filter angeordnet. Dem Filter sind vorteilhaft mindestens zwei Ablaufpfade nachgeschaltet. Gemäß einer Ausführung zweigt ein Seitenkanal vorteilhaft zwischen der Mikropumpe und dem Filter ab. Der Seitenkanal kann vorteilhaft einen Bypass zu dem Filter darstellen. Der Filter ist gemäß einer weiteren vorteilhaften Ausführungsform zwischen zwei Mikropumpen angeordnet.

Weitere Vorteile, Merkmale und Einzelheiten der Erfindung ergeben sich aus der nachfolgenden Beschreibung, in der unter Bezugnahme auf die Zeichnung verschiedene Ausführungsbeispiele im Einzelnen beschrieben sind.

### Kurze Beschreibung der Zeichnung

Es zeigen:
- Figur 1: eine vereinfachte Darstellung eines mikrofluidischen Systems mit einem Kanalnetz, in welchem eine Mikropumpe und eine Festphase angeordnet sind, gemäß einem ersten Ausführungsbeispiel;
- Figur 2: ein ähnliches mikrofluidisches System wie in Figur 1 gemäß einem zweiten Ausführungsbeispiel mit einem Seitenkanal, der die Festphase umgeht; und
- Figur 3: ein ähnliches mikrofluidisches System wie in den Figuren 1 und 2 mit einer zweiten Mikropumpe.

### Beschreibung der Ausführungsbeispiele

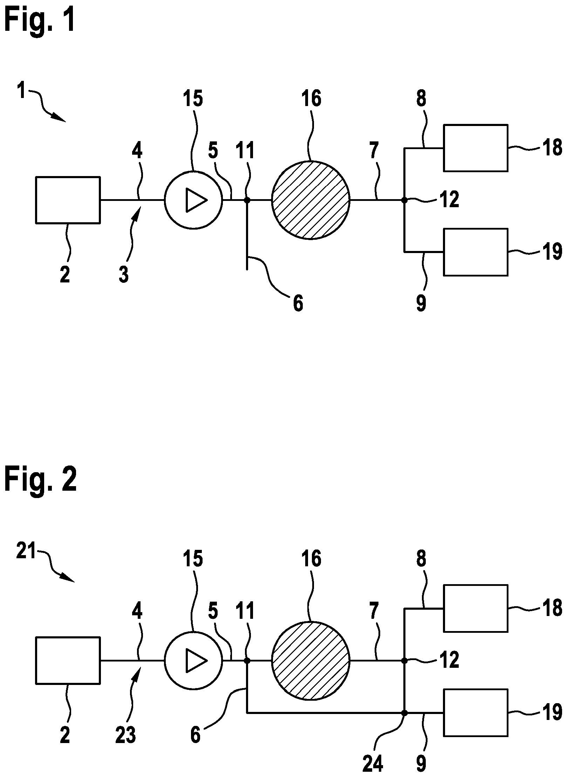

In den Figuren 1 bis 3 sind drei Ausführungsbeispiele eines mikrofluidischen Systems 1; 21; 31 zur Desorption von Nukleinsäuren von einer Festphase vereinfacht dargestellt. Das mikrofluidische System 1; 21; 31 umfasst ein Vorratsreservoir 2, das über ein mikrofluidische Kanalnetz 3; 23; 33 mit einer Mikropumpe 15 und einer Festphase 16 verbunden ist.

Bei den Nukleinsäuren handelt es sich zum Beispiel um Ribo-Nukleinsäuren (RNA) oder Desoxyribo-Nukleinsäuren (DNA). Bei der Festphase handelt es sich vorzugsweise um einen Filter, zum Beispiel einen Gewebe- oder Silika-Filter, mit einem Durchmesser zwischen einem und fünfundzwanzig Millimeter, insbesondere zwischen drei und vier Millimetern.

Die Nukleinsäuren werden aus einer lysierten Probe desorbiert. Bei der Probe handelt es sich um eine Flüssigkeit, die Zielzellen enthält, zum Beispiel eine Zellsuspension und/der eine Patientenprobe.

Die Patientenprobe umfasst zum Beispiel Blut, Lavage, Urin, Liquor, Sputum oder einen ausgespülten Swab beziehungsweise Abstrich. Das Volumen der Probe beträgt zwischen einigen Mikrolitern und zehn Millilitern, insbesondere zwischen 0,2 und einem Milliliter.

Bei der Mikropumpe 15 handelt es sich zum Beispiel um eine peristaltische Pumpe, eine Membranpumpe oder eine integrierte mikrofluidische Pumpe. Als Fraktion wird ein definiertes Volumen bezeichnet, das, beispielsweise durch eine integrierte mikrofluidische Pumpe, in das mikrofluidische Kanalsystem oder Kanalnetz 3; 23; 33 verdrängt wird.

Zur Vorbereitung der Desorption werden vorzugsweise mindestens ein Lyseschritt, ein Bindeschritt und ein Waschschritt durchgeführt. In dem Lyseschritt wird eine Probe, welche die nachzuweisenden Zielzellen enthält, mittels geeigneter Verfahren lysiert. Dabei werden die Zielzellen lysiert und die Nukleinsäuren freigesetzt. Das entstandene Gemisch wird als Lysat bezeichnet.

In dem optionalen Bindeschritt wird dem Lysat ein Bindepuffer hinzugegeben und mit dem Lysat vermischt. Anschließend wird das Gemisch über die Festphase 16, insbesondere den Silika-Filter, geleitet. Dabei erfolgt eine Adsorption der Nukleinsäuren an die Festphase beziehungsweise den Filter 16.

In dem Waschschritt wird mindestens ein oder werden mehrere Waschpuffer über die Festphase beziehungsweise den Filter 16 geleitet. In diesem Schritt bleiben die Nukleinsäuren an den Filter 16 gebunden, während beispielsweise Proteine oder Substanzen des Bindepuffers entfernt werden.

In einem anschließenden Elutionsschritt werden die Nukleinsäuren mit einem geeigneten Elutionspuffer von der Festphase beziehungsweise dem Silika-Filter 16 eluiert. Die Elution erfolgt vorteilhaft durch die in das mikrofluidische System 1 integrierte Mikropumpe 15. Dabei erfolgt die Elution mit Hilfe eines definierten Volumens, das im Folgenden als Fraktion bezeichnet wird, des Elutionspuffers, und das durch die Mikropumpe 15 verdrängt und über den Filter 16 geleitet wird. Dieser Schritt kann gemäß einer vorteilhaften Variante mehrmals erfolgen. Die Anströmrichtung kann in einer weiteren Ausführungsform umgedreht werden und somit das Eluat mehrmals über die Festphase 16 gegeben werden.

In den Figuren 1 bis 3 umfasst das mikrofluidische Kanalnetz 3; 23; 33 mikrofluidische Kanäle 4, 5, 6, 7, 8, 9. Die mikrofluidischen Kanäle 4 bis 9 werden verkürzt auch als Kanäle bezeichnet.

Der Kanal 4 verbindet den Vorratsbehälter 2 mit der Mikropumpe 15. Der Kanal 5 verbindet die Mikropumpe 15 mit der Festphase beziehungsweise dem Filter 16. In dem Kanal 5 ist eine Verbindungsstelle oder Verzweigungsstelle 11 angeordnet, von der ein Kanal 6 ausgeht, der als Seitenkanal 6 bezeichnet wird.

Ein Kanal 7 verbindet die Festphase beziehungsweise den Filter 16 mit einer Verbindungsstelle oder Verzweigungsstelle 12, von der die Kanäle 8 und 9 ausgehen. Durch eine Rechteck 18 am Ende des Kanals 8 ist ein Ablaufpfad 18 angedeutet. Durch ein Rechteck 19 am Ende des Kanals 9 ist ein Ablaufpfad 19 angedeutet.

Bei dem in Figur 1 dargestellten mikrofluidischen System 1 wird nach dem Waschen des Filters 16 mittels der Mikropumpe 15, die verkürzt auch als Pumpe bezeichnet wird, ein Elutionsmedium, zum Beispiel Wasser, über das Kanalnetzwerk 3 aus dem Vorratsreservoir 2 angesaugt. Eine erste Fraktion des Elutionsmediums wird über den Filter 16 in den ersten Ablaufpfad 18 gepumpt. In dem mikrofluidischen System 1 können sich auch weitere Kanäle und Reservoire, zum Beispiel zur Bevorratung weiterer Reagenzien oder zur Aufnahme von Reagenzienabfällen, befinden.

Im Anschluss wird eine zweite Fraktion des Elutionsmediums über den Filter 16 in den zweiten Ablaufpfad 19 gepumpt. Im zweiten Ablaufpfad 19 wird diese zweite eluierte Fraktion zum Beispiel von einer Kammer aufgenommen und weiter prozessiert. Ein Umschalten zwischen dem ersten Ablaufpfad 18 und dem zweiten Ablaufpfad 19 erfolgt zum Beispiel mittels (nicht dargestellter) mikrofluidischer Ventile an der Verbindungsstelle oder Verzweigungsstelle 12, die auch als Kanalkreuzung bezeichnet wird. Gegebenenfalls können auch weitere Elutionsfraktionen in weitere Ablaufpfade gepumpt werden.

In einer in Figur 1 dargestellten Variante des Kanalnetzwerks 3 geht vor dem Filter 16 der Seitenkanal 6 ab. Dieser Seitenkanal 6 fungiert in der Phase, während der die Mikropumpe 15 Elutionsmedium aus dem Vorratsreservoir 2 ansaugt, vorteilhaft als Ablaufkanal der Mikropumpe 15. Dadurch wird die Mikropumpe 15 vor dem eigentlichen Elutionsvorgang vollständig mit Elutionsmedium befüllt. Das liefert den Vorteil, dass bereits bei der Elution der ersten Fraktion exakt das Pumpenkammervolumen bewegt wird.

Gemäß einer ebenfalls in Figur 1 angedeuteten Prozessierungsvariante wird für die Elution einer bestimmten Fraktion das Elutionsmedium über den Filter 16 hin- und hergepumpt. Hierzu wird mit der Mikropumpe 15 die Elutionsfraktion zunächst über den Filter 16 in Richtung eines Ablaufpfades 18, 19 verdrängt. Die Elutionsfraktion wird dann wieder über den Filter 16 angesaugt. Diese Abfolge wird gegebenenfalls mehrfach, zum Beispiel dreimal oder fünfmal, wiederholt. Das liefert den Vorteil, dass eine besonders gute Durchmischung der Elutionsfraktion erfolgt und Nukleinsäuren besonders vollständig vom Filter 16 gelöst werden.

Gemäß einer weiteren Prozessierungsvariante werden die in den beiden Ablaufpfaden 18, 19 enthaltenen Fraktionen nach der Elution vermischt, zum Beispiel durch Hin- und Herpumpen zwischen in den jeweiligen Ablaufpfaden 18, 19 gelegenen Kammern mittels einer weiteren Pumpe (in Figur 1 nicht dargestellt). In den Ablaufpfaden 18, 19 können sich auch direkt Pumpkammern zur Aufnahme der Eluatfraktionen befinden, die zum Weitertransport der Eluatfraktionen genutzt werden können. Diese Prozessierungsvariante hat den Vorteil, dass beide Fraktionen effektiv vermischt werden und so die Konzentration von Inhibitoren herabgesetzt wird.

Bei dem in Figur 2 dargestellten mikrofluidischen System 21 umfasst ein Kanalnetz 23 im Vergleich zu dem Kanalnetz 3 aus Figur 1 eine zusätzliche Verbindungsstelle oder Verzweigungsstelle 24, die auch als Kanalkreuzung bezeichnet wird. Der Seitenkanal 6 erstreckt sich in dem Kanalnetz 23 zwischen den Verbindungs- oder Verzweigungsstellen 11 und 24. So kann der Seitenkanal 6 vorteilhaft genutzt werden, um den Filter 16 zu umgehen. Der Seitenkanal 6 mündet hinter dem Filter 16 in den Kanal 9 mit dem Ablaufpfad 19. Diese Variante hat den Vorteil, dass das Elutionsmedium über den Filter 16 zirkuliert werden kann. Hierdurch lässt sich eine besonders gute Durchmischung der Fraktionen erreichen.

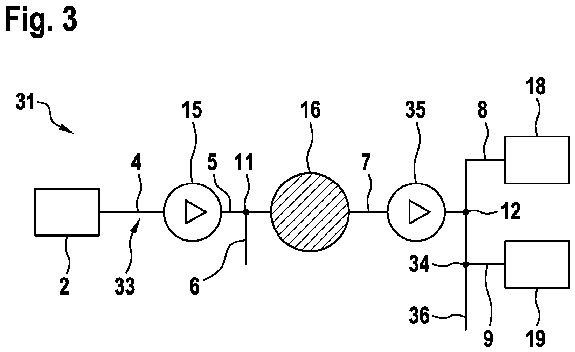

Das in Figur 3 dargestellte mikrofluidische System 31 umfasst ein Kanalnetz 33 mit einer zusätzlichen Verbindungsstelle oder Verzweigungsstelle 34. Die zusätzliche Verbindungsstelle oder Verzweigungsstelle 34 ist in dem Kanal 9 zu dem Ablaufpfad 19 angeordnet. Von der zusätzlichen Verbindungsstelle oder Verzweigungsstelle 34 geht ein weiterer Ablaufpfad 36 aus. Darüber hinaus ist in Figur 3 eine zweite Mikropumpe oder Pumpkammer 35 in dem Kanal 7 zwischen dem Filter 16 und der Verbindungsstelle oder Verzweigungsstelle 34 angeordnet. Mit der zweiten Mikropumpe oder Pumpkammer 35 kann das Hin- und Herpumpen in beide Richtungen aktiv unterstützt werden.

## Patentansprüche

1. Verfahren zur Desorption von Nukleinsäuren aus einer Probe, wobei eine Festphase (16) in einem mikrofluidischen System (1;21;31) mehrfach mit einem Elutionspuffer gespült wird, um an die Festphase (16) gebundene Nukleinsäuren in dem mikrofluidischen System (1;21;31) von der Festphase (16) zu eluieren, **dadurch gekennzeichnet, dass** Teilvolumina des Elutionspuffers seriell nacheinander über die Festphase (16) geleitet werden, wobei das erste über die Festphase geleitete Teilvolumen verworfen wird, vorzugsweise über Leitung in eine Auffangkammer des mikrofluidischen Systems (1;21;31).

2. Verfahren nach Anspruch 1, **dadurch gekennzeichnet, dass** die Elution durch eine in das mikrofluidische System (1;21;31) integrierte Mikropumpe (15) mit einem definierten Verdrängungsvolumen erfolgt, das einem Elutionsvolumen entspricht.

3. Verfahren nach Anspruch 2, **dadurch gekennzeichnet, dass** das Elutionsvolumen 1 bis 100 Mikroliter beträgt.

4. Verfahren nach einem der vorhergehenden Ansprüche, **dadurch gekennzeichnet, dass** ein Elutionsmedium in dem mikrofluidischen System (1;21;31) über die Festphase (16) hin- und hergepumpt wird.

5. Verfahren nach einem der vorhergehenden Ansprüche, **dadurch gekennzeichnet, dass** eine Anströmrichtung der Festphase (16) in dem mikrofluidischen System (1;21;31) umgekehrt wird.

6. Verfahren nach einem der vorhergehenden Ansprüche, **dadurch gekennzeichnet, dass** eine Eluatfraktion n und eine weitere Eluatfraktion n+1 in dem mikrofluidischen System (1;21;31) in einem Gemisch vereint werden, aus dem ein Aliquot für die weitere Analyse entnommen wird.

7. Verfahren nach einem der vorhergehenden Ansprüche, **dadurch gekennzeichnet, dass** vor der Elution mindestens einer, mehrere oder jeder der folgenden vorbereitenden Schritte in dem mikrofluidischen System (1;21;31) ausgeführt wird beziehungsweise werden:
- die Probe wird vor der Elution in dem mikrofluidischen System (1;21;31) lysiert, um die Zielzellen zu lysieren und die Nukleinsäuren freizusetzen;
- dem Lysat wird in dem mikrofluidischen System (1;21;31) in einem Bindeschritt ein Bindepuffer hinzugegeben, der mit dem Lysat vermischt wird;
- das Lysat beziehungsweise das in dem Bindeschritt entstandene Gemisch wird in dem mikrofluidischen System (1;21;31) über die Festphase (16) geleitet.

8. Mikrofluidisches System (1;21;31) mit einem Kanalnetz (3), in welchem eine Festphase (16) und mindestens eine Mikropumpe (15,35) angeordnet sind, **dadurch gekennzeichnet, dass** das mikrofluidische System (1;21;31) konfiguriert ist, um das Verfahren nach einem der vorhergehenden Ansprüche auszuführen, wobei die Mikropumpe (15,35) so in dem Kanalnetz (3) angeordnet und dazu eingerichtet ist, die Festphase (16) in dem mikrofluidischen System (1;21;31) mehrfach mit einem Elutionspuffer zu spülen und dabei Teilvolumina des Elutionspuffers seriell nacheinander über die Festphase zu leiten, wobei das erste über die Festphase (16) geleitete Teilvolumen verworfen wird, um Nukleinsäuren aus einer Probe zu desorbieren.

9. Mikrofluidisches System nach Anspruch 8, **dadurch gekennzeichnet, dass** die Festphase (16) ein in dem Kanalnetz (3) des mikrofluidischen Systems (1;21;31) angeordneter Filter ist, der über einen Verbindungskanal (5) mit der Mikropumpe (15,35) verbunden ist.

## Claims

1. Method for the desorption of nucleic acids from a sample, wherein a solid phase (16) is rinsed multiple times with an elution buffer in a microfluidic system (1; 21; 31) in order to elute nucleic acids bound to the solid phase (16) from the solid phase (16) in the microfluidic system (1; 21; 31), **characterized in that** part-volumes of the elution buffer are passed across the solid phase (16) one after another in series, the first part volume passed across the solid phase being discarded, preferably by being conducted into a collection chamber of the microfluidic system (1; 21; 31) .

2. Method according to Claim 1, **characterized in that** the elution is effected by a micropump (15) integrated into the microfluidic system (1; 21; 31) and having a defined displacement volume that corresponds to an elution volume.

3. Method according to Claim 2, **characterized in that** the elution volume is 1 to 100 microlitres.

4. Method according to any of the preceding claims, **characterized in that** an elution medium is pumped back and forth across the solid phase (16) in the microfluidic system (1; 21; 31).

5. Method according to any of the preceding claims, **characterized in that** a direction of incoming flow onto the solid phase (16) in the microfluidic system (1; 21; 31) is reversed.

6. Method according to any of the preceding claims, **characterized in that** an eluate fraction n and a further eluate fraction n+1 are combined in the microfluidic system (1; 21; 31) to form a mixture from which an aliquot is withdrawn for further analysis.

7. Method according to any of the preceding claims, **characterized in that**, prior to the elution, at least one, a plurality or each of the following preparatory steps is/are implemented in the microfluidic system (1; 21; 31) :
- the sample is lysed prior to the elution in the microfluidic system (1; 21; 31) in order to lyse the target cells and release the nucleic acids;
- a binding buffer is added to the lysate in the microfluidic system (1; 21; 31) in a binding step and is mixed with the lysate;
- the lysate or the mixture formed in the binding step is passed across the solid phase (16) in the microfluidic system (1; 21; 31).

8. Microfluidic system (1; 21; 31) having a channel network (3) in which a solid phase (16) and at least one micropump (15, 35) are arranged, **characterized in that** the microfluidic system (1; 21; 31) is configured to implement the method according to any of the preceding claims, wherein the micropump (15, 35) is arranged in the channel network (3) and set up so as to rinse the solid phase (16) multiple times with an elution buffer in the microfluidic system (1; 21; 31) and in the process to pass part-volumes of the elution buffer across the solid phase one after another in series, the first part volume passed across the solid phase (16) being discarded, in order to desorb nucleic acids from a sample.

9. Microfluidic system according to Claim 8, **characterized in that** the solid phase (16) is a filter which is arranged in the channel network (3) of the microfluidic system (1; 21; 31) and is connected to the micropump (15, 35) via a connecting channel (5).

## Revendications

1. Procédé pour la désorption d'acides nucléiques à partir d'un échantillon, dans lequel une phase solide (16) dans un système microfluidique (1;21;31) est lavée plusieurs fois avec un tampon d'élution, afin d'éluer de la phase solide (16) des acides nucléiques liés à la phase solide (16) dans le système microfluidique (1;21;31), **caractérisé en ce que** des volumes partiels du tampon d'élution sont envoyés en série successivement sur la phase solide (16), le premier volume partiel envoyé sur la phase solide étant rejeté, de préférence via un conduit dans une chambre collectrice du système microfluidique (1;21;31).

2. Procédé selon la revendication 1, **caractérisé en ce que** l'élution s'effectue au moyen d'une micropompe (15) intégrée dans le système microfluidique (1;21;31), ayant un volume de déplacement défini qui correspond à un volume d'élution.

3. Procédé selon la revendication 2, **caractérisé en ce que** le volume d'élution vaut de 1 à 100 microlitres.

4. Procédé selon l'une quelconque des revendications précédentes, **caractérisé en ce qu'**un milieu d'élution est pompé en va-et-vient sur la phase solide (16) dans le système microfluidique (1;21;31).

5. Procédé selon l'une quelconque des revendications précédentes, **caractérisé en ce qu'**une direction d'écoulement sur la phase solide (16) est inversée dans le système microfluidique (1;21;31).

6. Procédé selon l'une quelconque des revendications précédentes, **caractérisé en ce qu'**une fraction n d'éluat et une autre fraction n+1 d'éluat dans le système microfluidique (1;21;31) sont réunies en un mélange, à partir duquel une partie aliquote est prélevée pour l'analyse ultérieure.

7. Procédé selon l'une quelconque des revendications précédentes, **caractérisé en ce qu'**avant l'élution au moins une, plusieurs ou chacune des étapes préparatoires suivantes est ou sont effectuée(s) dans le système microfluidique (1;21;31) :
- avant l'élution l'échantillon est lysé dans le système microfluidique (1;21;31), afin de lyser les cellules cibles et de libérer les acides nucléiques ;
- dans une étape de liaison, au lysat dans le système microfluidique (1;21;31) est ajouté un tampon de liaison, qui est mélangé avec le lysat ;
- le lysat ou le mélange formé dans l'étape de liaison est envoyé sur la phase solide (16) dans le système microfluidique (1;21;31).

8. Système microfluidique (1;21;31) comportant un réseau (3) de canaux, dans lequel sont disposées une phase solide (16) et au moins une micropompe (15,35), **caractérisé en ce que** le système microfluidique (1;21;31) est configuré pour exécuter le procédé selon l'une quelconque des revendications précédentes, la micropompe (15,35) étant disposée de telle façon dans le réseau (3) de canaux et conçue pour laver plusieurs fois avec un tampon d'élution la phase solide (16) dans le système microfluidique (1;21;31) et ce faisant envoyer en série successivement sur la phase solide des volumes partiels du tampon d'élution, le premier volume partiel envoyé sur la phase solide (16) étant rejeté; afin de désorber d'un échantillon des acides nucléiques.

9. Système microfluidique selon revendication 8, **caractérisé en ce que** la phase solide (16) est un filtre disposé dans le réseau (3) de canaux du système microfluidique (1;21;31), qui est relié par un canal de raccordement (5) à la micropompe (15,35).
